# EUROPÄISCHE PATENTANMELDUNG

(11) **EP 0 931 552 A2**
(43) Veröffentlichungstag der Anmeldung: **28.07.1999**
(21) Anmeldenummer: 99100495.3
(22) Anmeldetag: 12.01.1999
(51) Int. Cl.: A61K 47/34, A61K 9/16, A61K 9/20, A61K 9/24

(54) **Verfahren zur Herstellung von festen Dosierungsformen**

(30) Priorität: 13.01.1998 DE 19800927
(71) Anmelder: BASF AKTIENGESELLSCHAFT, 67056 Ludwigshafen (DE)
(72) Erfinder: Kothrade, Stephan, 67117 Limburgerhof (DE); Müller, Wolfgang, 67227 Frankenthal (DE); Berndl, Gunther, 67273 Herxheim (DE); Sanner, Axel, 67227 Frankenthal (DE)
(74) Vertreter: Kinzebach, Werner, Dr.

(57) **Zusammenfassung**

Gegenstand der vorliegenden Erfindung ist ein Verfahren zur Herstellung von festen Dosierungsformen durch Vermischen von mindestens einem polymeren Bindemittel, gegebenenfalls mindestens einem Wirkstoff und gegebenenfalls üblichen Additiven unter Bildung eines plastischen Gemisches, wobei als polymeres Bindemittel sulfonatgruppentragende Polyamide verwendet werden.

## Beschreibung

Die Erfindung betrifft ein Verfahren zur Herstellung von festen Dosierungsformen durch Vermischen von mindestens einem polymeren Bindemittel und gegebenenfalls mindestens einem Wirkstoff und gegebenenfalls üblichen Additiven unter Bildung eines plastischen Gemisches und Formgebung. Insbesondere betrifft die Erfindung ein Verfahren zur Herstellung von festen pharmazeutischen Formen.

Die klassischen Verfahren zur Herstellung fester pharmazeutischer Formen, insbesondere Tabletten, werden diskontinuierlich durchgeführt und umfassen mehrere Stufen. Pharmazeutische Granulate stellen hierbei ein wichtiges Zwischenprodukt dar. So ist z. B. dem Buch "Pharmazeutische Technologie", Verfasser Prof. Bauer, Frömmig und Führer, Thieme Verlag, Seiten 292 ff., zu entnehmen, dass man Arzneiformen über Trockengranulierung aus der Schmelze gewinnen kann. Es wird beschrieben, dass Schmelzerstarrungsgranulate entweder durch Schmelzen und Schockerstarren, durch Ausgießen und Zerkleinern oder durch Sprüherstarren in Sprühtürmen hergestellt werden können. Ein Problem bei diesen Verfahren ist die für die Herstellung von Arzneimitteln erforderliche exakte Formgebung. Es werden häufig unregelmäßige Partikel oder Bruchstücke erzeugt, so dass die erzielte Form in keiner Weise den üblichen Arzneiformen entspricht und Granulate deshalb als eigenständige Arzneiform nur eine geringe Bedeutung besitzen. Die Herstellung der gewünschten festen Arzneiformen erfordert den Einsatz weiterer Verfahrensschritte, wie zum Beispiel die Komprimierung mit Hilfe von Tablettiermaschinen. Dies ist zeit- und kostenintensiv.

Seit einiger Zeit ist ein wesentlich einfacheres kontinuierliches Verfahren zur Herstellung fester pharmazeutischer Formen bekannt, bei dem man eine wirkstoffhaltige, lösungsmittelfreie Schmelze aus einem polymeren, wirkstoffhaltigen Bindemittel extrudiert und den extrudierten Strang zu der gewünschten Arzneiform formt, beispielsweise in einem Kalander mit Formwalzen, siehe EP-A-240 904, EP-A-240 906 und EP-A-337 256 und EP-A-358105. Damit kann eine gezielte Formgebung erreicht werden. Als polymeres Bindemittel werden insbesondere Polymere des N-Vinylpyrrolidons oder Copolymerisate davon, z. B. mit Vinylacetat, eingesetzt.

Die Verwendung von Polyamiden in der Medizintechnik und der Pharmazie ist dem Fachmann bekannt. Implantate, die transdermale Applikation von Arzneistoffen, Dialysemembrane und Pharmacoatings stellen nur einige Beispiele aus den vielfältigen Anwendungsbereichen von Polyamiden dar.

Die WO 96/21427 Al beschreibt beispielsweise, dass Polyamide als biologisch abbaubare, wasserunlösliche, polyamidhaltige Copolymere in flüssigen Arzneimittelformulierungen mit kontrollierter Wirkstofffreisetzung eingesetzt werden können. Diese Polymere enthalten den Wirkstoff in gelöster, dispergierter oder suspendierter Form.

Die Verwendung von Polyamiden in festen pharmazeutischen Zusammensetzungen wird in der WO 93/24154 A1 erwähnt. Diese Zusammensetzungen basieren auf schmelzgesponnenen Polymeren, deren überwiegend amorpher Charakter für eine rasche und gleichbleibende Wirkstofffreisetzung sorgen soll.

Ein Verfahren zur Herstellung von Arzneipillen und Tabletten wird in der DE-OS-1 766 546 beschrieben. Es handelt sich dabei um einen Formgebungsvorgang, bei dem die Trägersubstanz in eine den Wirkstoff in verteilter und/oder gelöster Form enthaltende Schmelze überführt wird, diese Schmelze in den trogähnlichen Raum eines rotierenden Walzenpaares gebracht wird, wobei die Walzen an ihren Mantelflächen mit Hohlräumen zur Aufnahme der Dragiermasse versehen und gekühlt sind, um die flüssige Dragiermasse in Hohlräumen erstarren zu lassen, und die so erhaltenen Arzneimittel die Hohlräume verlassen. Die Trägersubstanz kann auch Thermoplasten umfassen, wie Polyvinylchlorid, Polyethylen, Polypropylen, Polyamide, Polystyrol, Polyvinylidenchlorid, Acrylnitril-Butadien-Styrol oder Acrylnitril-Styrol.

Polyamidhaltige Zusammensetzungen mit verzögerter Wirkstofffreisetzung werden in der EP 381 445 A2 und der EP 381 446 A1 erwähnt. Es handelt sich dabei um topische Mittel zur Zahn- bzw. Mundbehandlung, bei denen der jeweilige Wirkstoff in Cellulose oder einem hydrophoben Polymer eingebettet ist.

Sulfonatgruppentragende Polymere werden in der DE 40 37 518 A1 als geeignete Komponente zur Herstellung bestimmter Harzteilchen mit enger Größenverteilung und im Wesentlichen kugeliger Gestalt beschrieben. Derartige Harzpartikel sind insbesondere für elektrofotografische Toner brauchbar. Eine Verwendung als Arzneimittelträger wird beiläufig erwähnt.

Die Herstellung obiger Zusammensetzungen ist jedoch vielfach sehr aufwendig und damit zeit- und kostenintensiv und führt in den meisten Fällen zu Dosierungsformen mit rascher Freisetzungscharakteristik.

Aufgabe der Erfindung war es daher, ein einfaches und kostengünstiges Verfahren zur Herstellung von festen Dosierungsformen, insbesondere Arzneiformen, mit verzögerter Wirkstofffreisetzung zur Verfügung zu stellen.

Überraschenderweise wurde nun gefunden, dass diese Aufgabe gelöst wird, wenn man die Dosierungsformen durch Schmelzextrusion herstellt und dabei sulfonatgruppentragende Polyamide als Bindemittel verwendet.

Gegenstand der vorliegenden Erfindung ist daher ein Verfahren zur Herstellung von festen Dosierungsformen durch Vermischen von mindestens einem polymeren Bindemittel, gegebenenfalls mindestens einem Wirkstoff und gegebenenfalls üblichen Additiven unter Bildung eines plastischen Gemisches und Formgebung, dadurch gekennzeichnet, dass sulfonatgruppentragende Polyamide als polymeres Bindemittel verwendet werden.

Das erfindungsgemäße Verfahren ermöglicht die Herstellung von festen Dosierungsformen auf einfache und kostengünstige Weise. Die vorteilhaften Eigenschaften der sulfonatgruppentragenden Polyamide werden durch die Überführung in den plastischen Zustand nicht beeinträchtigt. Darüber hinaus ergibt das erfindungsgemäße Verfahren überraschenderweise Dosierungsformen mit sehr langsamer Wirkstofffreisetzung (Retard-Formulierungen), wohingegen die bisher verwendeten Polyamide Dosierungsformen mit rascher Wirkstofffreisetzung ergaben. Über Abmischungen mit schneller freisetzenden Hilfsstoffen können so beliebige Freisetzungsprofile erzielt werden. Außerdem gelingt aufgrund der hohen Glasübergangstemperatur die Herstellung harter, klebfreier Darreichungsformen, die sich selbst bei hohen Temperaturen durch eine gute Lagerstabilität auszeichnen.

Unter Dosierungsformen sind hier alle Formen zu verstehen, die zur Verwendung als Arzneimittel, Pflanzenbehandlungsmittel, Futtermittel und Nahrungsmittel und zur Abgabe von Riechstoffen und Parfümölen geeignet sind. Dazu gehören beispielsweise Tabletten jeglicher Form, Pellets, Granulate, aber auch größere Formen, wie Würfel, Blöcke (Quader) oder zylindrische Formen, die sich insbesondere als Futter- oder Nahrungsmittel verwenden lassen.

Die erfindungsgemäß erhältlichen Dosierungsformen umfassen im Allgmeinen:
a) 0 bis 90 Gew.-%, insbesondere 0,1 bis 60 Gew.-% (bezogen auf das Gesamtgewicht der Dosierungsform) eines Wirkstoffes,
b) 10 bis 100 Gew.-%, insbesondere 40 bis 99,9 Gew.-% eines polymeren Bindemittels und
c) gegebenenfalls Additive.

Wenn die Dosierungsform für Nahrungsmittelzwecke oder Futtermittelzwecke eingesetzt wird, kann der Wirkstoff fehlen, d. h. die Dosierungsform kann bis zu 100 % des polymeren Bindemittels umfassen.

Als polymeres Bindemittel werden erfindungsgemäß sulfonatgruppentragende Polyamide verwendet. Die Sulfonatgruppen können mit einem oder mehreren der zum Aufbau der Polyamide eingesetzten Monomere eingeführt werden. Dabei können diese Monomere eine oder mehrere Sulfonatgruppen aufweisen. Der molare Anteil an sulfonatgruppentragenden Monomeren beträgt in der Regel wenigstens 0,5 mol-% und vorzugsweise nicht mehr als 50 mol-%. Besonders bevorzugt sind 5 bis 35 mol-% und insbesondere 10 bis 30 mol-%.

Sulfonatgruppentragende Dicarbonsäuren eignen sich besonders gut zum Aufbau der erfindungsgemäß eingesetzten Polyamide. Werden neben diesen sulfonatgruppentragende Dicarbonsäuren weitere Dicarbonsäuren verwendet, so beträgt das Verhältnis der molaren Anteile von sulfonatgruppentragenden Dicarbonsäuren zu weiteren Dicarbonsäuren in der Regel 1:99 bis 99:1, vorzugsweise 10:90 bis 70:30 und insbesondere 20:80 bis 60:40.

Vorzugsweise werden sulfonatgruppentragende Polyamide verwendet, die erhältlich sind aus
A1) 0 bis 90 mol-% mindestens einer Monoaminocarbonsäure, deren Lactam oder Monoaminocarbonsäure-/-lactam-Gemischen,
A2) 5 bis 50 mol-% mindestens eines primären oder sekundären Diamins,
A3) 0,5 bis 49,5 mol-% mindestens einer sulfonatgruppentragenden Dicarbonsäure und gegebenenfalls
A4) 0,5 bis 49,5 mol-% mindestens einer weiteren Dicarbonsäure, wobei die Summe der molaren Anteile der Monomeren A3) und A4) im Wesentlichen dem molaren Anteil des Monomeren A2) entspricht.

Besonders bevorzugt ist die Verwendung sulfonatgruppentragender Polyamide, die erhältlich sind aus
A1) 0 bis 50 mol-% mindestens einer Monoaminocarbonsäure, deren Lactam oder Monoaminocarbonsäure-/-lactam-Gemischen,
A2) 45 bis 50 mol-% mindestens eines primären oder sekundären Diamins,
A3) 5 bis 35 mol-% mindestens einer sulfonatgruppentragenden Dicarbonsäure und gegebenenfalls
A4) 15 bis 45 mol-% mindestens einer weiteren Dicarbonsäure, wobei die Summe der molaren Anteile der Monomeren A3) und A4) im Wesentlichen dem molaren Anteil des Monomeren A2) entspricht.

Ganz besonders bevorzugt sind sulfonatgruppentragende Polyamide, die erhältlich sind aus
A1) 0 bis 45 mol-% mindestens einer Monoaminocarbonsäure, deren Lactam oder Monoaminocarbonsäure-/-lactam-Gemischen,
A2) 47,5 bis 50 mol-% mindestens eines primären oder sekundären Diamins,
A3) 10 bis 30 mol-% mindestens einer sulfonatgruppentragenden Dicarbonsäure und gegebenenfalls
A4) 20 bis 40 mol-% mindestens einer weiteren Dicarbonsäure, wobei die Summe der molaren Anteile der Monomeren A3) und A4) im Wesentlichen dem molaren Anteil des Monomeren A2) entspricht.

Wenn vorhanden, beträgt der Anteil der Komponente A1 mindestens 0,5 mol-%.

Vorteilhaft sind Polyamide, die nur aus den oben beschriebenen Monomeren A2), A3) und A4) erhältlich sind. In diesem Fall beträgt der molare Anteil des Monomeren A2) 50 mol-%. Die molaren Anteile der Monomere A3) und A4) bewegen sich innerhalb der oben vorgegebenen Grenzen, wobei die Summe ebenfalls 50 mol-% beträgt.

Besonders vorteilhaft ist es, wenn mindestens zwei verschiedene Diamine zur Herstellung der erfindungsgemäßen Polymere verwendet werden.

Als Monomere A1) eignen sich die für die Herstellung von Polyamiden bekannten Monoaminocarbonsäuren und deren Lactame. Es handelt sich vorzugsweise um C₂-C₁₂-Monoaminocarbonsäuren und insbesondere um Monoaminocarbonsäuren der allgemeinen Formel H₂N-R¹-COOH, worin R¹ für einen geradkettigen oder verzweigten, gesättigten oder ungesättigten, aliphatischen, cycloaliphatischen oder aromatischen Rest steht. Derartige Reste R¹ können auch ein- oder mehrfach mit unabhängig aus Hydroxy oder C₁₋₄-Alkoxy ausgewählten Gruppen substituiert sein. Beispiele geeigneter Monoaminocarbonsäuren und Lactame sind ω-Aminoundecansäure, Pyrrolidon, ε-Caprolactam, Laurinlactam, Capryllactam oder Önantholactam.

Als Monomere A2) kommen beispielsweise primäre C₂-C₁₈-Diamine, insbesondere solche der Formel H₂N-R²-NH₂ in Betracht, worin R² für einen geradkettigen oder verzweigten, gesättigten und ungesättigten, aliphatischen Rest mit 2 bis 18, vorzugsweise mit 2 bis 14 und insbesondere mit 5 bis 11 Kohlenstoffatomen, der auch ein- oder mehrfach mit unabhängig aus Hydroxy oder C₁₋₄-Alkoxy ausgewählten Gruppen substituiert sein kann, einen gesättigten oder ungesättigten cycloaliphatischen Rest mit 5 bis 8 und vorzugsweise 6 Kohlenstoffatomen, der auch ein- oder mehrfach mit unabhängig aus Hydroxy, C₁₋₄-Alkyl oder C₁₋₄-Alkoxy ausgewählten Gruppen substituiert sein kann, mehrere und insbesondere zwei miteinander verknüpfte cycloaliphatische Reste der vorbezeichneten Art, einen aromatischen Rest mit 6 bis 18, vorzugsweise 6 bis 12 Kohlenstoffatomen und insbesondere einen Phenylrest, der auch ein- oder mehrfach mit unabhängig aus Hydroxy, C₁₋₄-Alkyl oder C₁₋₄-Alkoxy ausgewählten Gruppen substituiert sein kann, oder mehrere und insbesondere zwei miteinander verknüpfte aromatische Reste der vorbezeichneten Art steht. Aliphatische und cycloaliphatische Reste sind bevorzugt.

Miteinander verknüpfte, cycloaliphatische oder aromatische Reste sind entweder über eine Bindung oder einen divalenten Rest miteinander verknüpft, wobei der divalente Rest insbesondere eine C₁-C₄-Alkylenqruppe, wie eine Methylen-, 1,1,- und 1,2-Ethylen-, 1,1,- 1,2-, 1,3- und 2,2-Propylengruppe, -O-, -S-, -SO₂-, C(O) sowie eine durch -O-, -S-, -SO₂- oder C(O) unterbrochene Alkylengruppe der vorbezeichneten Art sein kann.

Bei aliphatischen Resten handelt es sich vorzugsweise um Alkylen- oder Alkenylengruppen, die auch einfach oder mehrfach durch -O-, -S-, -SO₂- oder C(O) unterbrochen sein können.

Bei cycloaliphatischen Resten handelt es sich vorzugsweise um Cycloalkyl- oder Cycloalkenylgruppen, die auch einfach oder mehrfach durch -O-, -S-, -SO₂- oder C(O) unterbrochen sein können.

Geeignete primäre Diamine sind beispielsweise Alkylendiamine oder Cycloalkyldiamine, wie 1,2-Ethandiamin, 1,5-Pentandiamin, Di(4-aminocyclohexyl)methan, 2,2'-Di(4-aminocyclohexyl)propan, Di(3-methyl-4-aminocyclohexyl)methan oder, vorzugsweise, Hexamethylendiamin. Weiterhin eignen sich auch 2,2,4-Trimethylhexamethylendiamin, 2-Butyl-2-ethyl-1,5-pentandiamin, 2-Methylpentamethylendiamin oder 4,7-Dioxadecan-1,10-diamin.

Als Monomere A2) eignen sich auch sekundäre Diamine, beispielsweise solche, die sich von einem primären Diamin der oben geschilderten Art durch Austausch wenigstens eines Aminwasserstoffs durch einen geeigneten Substituenten, wie C₁-C₃-Alkyl, ableiten.

Bevorzugte sekundäre Diamine sind cyclische Diamine der allgemeinen Formel 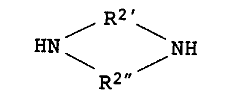 worin R^{2'} und R^{2"} unabhängig voneinander die oben für R² angegebenen Bedeutungen besitzen können. Ein Beispiel für ein solches Diamin ist Piperazin.

Als sulfonatgruppentragende Monomere A3) kommen solche in Betracht, in denen die Sulfonsäuregruppe als Salz vorliegt, z. B. als Salz eines Alkalimetalls, wie Lithium, Natrium oder Kalium, oder einer Ammoniumgruppe, die gegebenenfalls mit einer bis 4 aliphatischen oder aromatischen Gruppen substituiert ist. Geeignete sulfonatgruppentragende Monomere sind Sulfonsäuresalze von C₄-C₂₀- und vorzugsweise C₄-C₁₂-Dicarbonsäuren. Besonders geeignet sind Dicarbonsäuren der allgemeinen Formel HOOC-R³-COOH, worin R³ die oben für R² genannten Bedeutungen besitzen kann.

Vorzugsweise steht R³ für einen aromatischen Rest. Hierzu zählen Phenyl-, Naphthyl- oder Diphenylreste sowie zwei über einen divalenten Rest miteinander verknüpfte Phenylreste. Beispiele für derartige divalente Reste sind oben angegeben.

Die Sulfonatgruppen können direkt oder über eine C₁-C₄-Alkylenbrücke an R³ gebunden sein. Ein Beispiel für aliphatische, sulfonatgruppentragende Dicarbonsäuren ist die Sulfobernsteinsäure. Geeignete aromatische sulfonatgruppentragende Dicarbonsäuren basieren beispielsweise auf der Phthalsäure, Isophthalsäure, Terephthalsäure, 1,4- und 2,6-Naphthalindicarbonsäure, 3,3'- und 4,4'-Diphenyldicarbonsäure, 3,3'- und 4,4'-Diphenylmethandicarbonsäure oder der 2-Phenoxyterephthalsäure. In der Regel weisen die aromatischen Dicarbonsäuren eine oder zwei Sulfonatgruppen auf. Diese können an beliebige, nicht mit Carboxylgruppen substituierte Positionen direkt, beispielsweise wie in der 5-Sulfoisophthalsäure, oder über eine divalente Brücke, beispielsweise wie in der 5-Sulfopropoxyisophthalsäure, gebunden sein. Besonders bevorzugt wird ein Salz der 5-Sulfoisophthalsäure, insbesondere das Natriumsalz, eingesetzt.

Geeignete Monomere A4) sind vorzugsweise C₂-C₁₆-Dicarbonsäuren und insbesondere Dicarbonsäuren der allgemeinen Formel HOOC-R⁴-COOH. Für den Rest R⁴ gilt im Prinzip die Definition des Restes R³, außer dass R⁴ keine Sulfonatgruppe trägt. Beispiele aliphatischer Dicarbonsäuren sind Azelainsäure, Dodecandicarbonsäure oder, bevorzugt, Adipinsäure oder Sebacinsäure. Geeignete aromatische Dicarbonsäuren sind beispielsweise Isophthalsäure oder Terephthalsäure, die auch substituiert sein können, wie beispielsweise 3-tert.-Butylisophthalsäure, weiterhin 3,3'- oder 4,4'-Diphenyldicarbonsäure, 3,3'- oder 4,4'-Diphenylmethandicarbonsäure, 3,3'-oder 4,4'-Diphenylsulfondicarbonsäure, 1,4- oder 2,6-Naphthalindicarbonsäure oder 2-Phenoxyterephthalsäure.

Selbstverständlich gilt für alle Monomerengruppen, dass auch Gemische der jeweiligen Monomeren eingesetzt werden können.

Die Herstellung der sulfonatgruppentragenden Polyamide kann in an sich bekannter Weise erfolgen.

Als bevorzugte Herstellweise sei der Batch-Prozess (diskontinuierliche Herstellweise) genannt. Dabei wird die wässrige Monomerlösung innerhalb von 0,5 bis 3 h in einem Autoklaven auf Temperaturen von 240 bis 300 °C erhitzt, wobei ein Druck von 10 bis 50 bar, insbesondere 15 bis 30 bar, erreicht wird, der durch Entspannen von überschüssigem Wasserdampf bis zu 4 h konstant gehalten wird. Danach entspannt man den Autoklaven bei konstanter Temperatur innerhalb eines Zeitraumes von 0,5 bis 3 h auf Normaldruck. Anschließend wird die Polymerschmelze dem Autoklaven entnommen, mit Luft oder Stickstoff abgekühlt und anschließend granuliert.

Das so erhaltene Copolyamid hat in der Regel eine Viskositätszahl zwischen 25 und 110 ml/g, bevorzugt von 30 bis 80 ml/g, gemessen an einer 0,5 gew.-%igen Lösung in 96%iger Schwefelsäure.

Die Copolymerisate besitzen im Allgemeinen K-Werte von mindestens 7, vorzugsweise 10 bis 250. Die Polymere können K-Werte von bis zu 300 haben. Die Bestimmung der K-Werte erfolgt nach H. Fikentscher, Cellulosechemie, Band 13, 58-64 und 71-74 (1932), in wässriger Lösung oder in einem organischen Lösungsmittel bei 25 °C und bei Konzentrationen, die je nach K-Wert-Bereich zwischen 0,1 % und 5 % liegen.

Neben den oben beschriebenen polymeren Bindemitteln können, insbesondere bis zu 30 Gew.-%, bezogen auf das Gesamtgewicht des Bindemittels, an weiteren Bindemitteln eingesetzt werden, wie Polymere, Copolymere, Cellulosederivate, Stärke und Stärkederivate eingesetzt werden. Geeignet sind beispielsweise:

Polyvinylpyrrolidon (PVP), Copolymerisate von N-Vinylpyrrolidon (NVP) und Vinylestern, insbesondere Vinylacetat, Copolymerisate von Vinylacetat und Crotonsäure, teilverseiftes Polyvinylacetat, Polyvinylalkohol, Polyvinylformamid, partiell oder vollständig hydrolysiertes Polyvinylformamid, Polyhydroxyalkylacrylate, Polyhydroxyalkylmethacrylate, Polyacrylate und Polymethacrylate (Eudragit-Typen), Copolymerisate von Methylmethacrylat und Acrylsäure, Polyacrylamide, Polyethylenglykole, Celluloseester, Celluloseether, insbesondere Methylcellulose und Ethylcellulose, Hydroxyalkylcellulosen, insbesondere Hydroxypropylcellulose, Hydroxyalkyl-Alkylcellulosen, insbesondere Hydroxypropyl-Ethylcellulose, Cellulosephthalate, insbesondere Celluloseacetatphthalat und Hydroxypropylmethylcellulosephthalat, und Mannane, insbesondere Galactomannane. Davon sind Polyvinylpyrrolidon, Copolymerisate von N-Vinylpyrrolidon und Vinylestern, Polyhydroxyalkylacrylate, Polyhydroxyalkylmethacrylate, Polyacrylate, Polymethacrylate, Alkylcellulosen und Hydroxyalkylcellulosen besonders bevorzugt.

Das polymere Bindemittel muß in der Gesamtmischung aller Komponenten im Bereich von 50 bis 180 °C, vorzugsweise 60 bis 130 °C erweichen oder schmelzen. Die Glasübergangstemperatur der Mischung muss daher unter 180 °C, vorzugsweise unter 130 °C liegen. Erforderlichenfalls wird sie durch übliche, pharmakologisch akzeptable weichmachende Hilfsstoffe herabgesetzt. Die Menge an Weichmacher beträgt höchstens 30 Gew.-%, bezogen auf das Gesamtgewicht von Bindemittel und Weichmacher, damit lagerstabile Arzneiformen gebildet werden, die keinen kalten Fluss zeigen. Vorzugsweise aber enthält das Gemisch keinen Weichmacher.

Beispiele für derartige Weichmacher sind:
langkettige Alkohole, Ethylenglykol, Propylenglykol, Glycerin, Trimethylolpropan, Triethylenglykol, Butandiole, Pentanole, wie Pentaerythrit, Hexanole, Polyethylenglykole, Polypropylenglykole, Polyethylen-propylenglykole, Silicone, aromatische Carbonsäureester (z. B. Dialkylphthalate, Trimellithsäureester, Benzoesäureester, Terephthalsäureester) oder aliphatische Dicarbonsäureester (z. B. Dialkyladipate, Sebacinsäureester, Azelainsäureester, Zitronen- und Weinsäureester), Fettsäureester, wie Glycerinmono-, Glycerindi- oder Glycerintriacetat oder Natriumdiethylsulfosuccinat. Die Konzentration an Weichmacher beträgt im Allgemeinen 0,5 bis 15, vorzugsweise 0,5 bis 5 Gew.-%, bezogen auf das Gesamtgewicht des Gemisches.

Übliche galenische Hilfsstoffe, deren Gesamtmenge bis zu 100 Gew.-%, bezogen auf das Polymerisat, betragen kann, sind z. B. Streckmittel bzw. Füllstoffe, wie Silikate oder Kieselerde, Magnesiumoxid, Aluminiumoxid, Titanoxid, Stearinsäure oder deren Salze, z. B. das Magnesium- oder Calciumsalz, Methylcellulose, Natrium-Carboxymethylcellulose, Talkum, Saccharose, Lactose, Getreide- oder Maisstärke, Kartoffelmehl, Polyvinylalkohol, insbesondere in einer Konzentration von 0,02 bis 50, vorzugsweise 0,20 bis 20 Gew.-%, bezogen auf das Gesamtgewicht des Gemisches.

Schmiermittel, wie Aluminium- und Calciumstearat, Talkum und Silicone, in einer Konzentration von 0,1 bis 5, vorzugsweise 0,1 bis 3 Gew.-%, bezogen auf das Gesamtgewicht des Gemisches.

Fließmittel, wie tierische oder pflanzliche Fette, insbesondere in hydrierter Form und solche, die bei Raumtemperatur fest sind. Diese Fette haben vorzugsweise einen Schmelzpunkt von 50°C oder höher. Bevorzugt sind Triglyceride der C₁₂-, C₁₄-, C₁₆- und C₁₈-Fettsäuren. Auch Wachse, wie Carnaubawachs, sind brauchbar. Diese Fette und Wachse können vorteilhaft alleine oder zusammen mit Mono- und/oder Diglyceriden oder Phosphatiden, insbesondere Lecithin, zugemischt werden. Die Mono- und Diglyceride stammen vorzugsweise von den oben erwähnten Fettsäuretypen ab. Die Gesamtmenge an Fetten, Wachsen, Mono-, Diglyceriden und/oder Lecithinen beträgt 0,1 bis 30, vorzugsweise 0,1 bis 5 Gew.-%, bezogen auf das Gesamtgewicht der Masse für die jeweilige Schicht; Farbstoffe, wie Azofarbstoffe, organische oder anorganische Pigmente oder Farbstoffe natürlicher Herkunft, wobei anorganische Pigmente in einer Konzentration von 0,001 bis 10, vorzugsweise 0,5 bis 3 Gew.-%, bezogen auf das Gesamtgewicht des Gemisches bevorzugt sind;

Stabilisatoren, wie Antioxidanzien, Lichtstabilisatoren, Hydroperoxid-Vernichter, Radikalfänger, Stabilisatoren gegen mikrobiellen Befall.

Ferner können Netz-, Konservierungs-, Spreng-, Adsorptions-, Formentrenn-, Dispergierhilfs- und Treibmittel sowie Entschäumer zugesetzt werden (vgl. z. B. H. Sucker et al., Pharmazeutische Technologie, Thieme-Verlag, Stuttgart 1978).

Unter Hilfsstoffen im Sinne der Erfindung sind auch Substanzen zur Herstellung einer festen Lösung des Wirkstoffs zu verstehen. Diese Hilfsstoffe sind beispielsweise Pentaerythrit und Pentaerythrit-tetraacetat, Polymere wie z. B. Polyethylen- bzw. Polypropylenoxide und deren Blockcopolymere (Poloxamere), Phosphatide wie Lecithin, Homo- und Copolymere des Vinylpyrrolidons, Tenside wie Polyoxyethylen-40-stearat sowie Zitronen- und Bernsteinsäure, Gallensäuren, Sterine und andere wie z. B. bei J. L. Ford, Pharm. Acta Helv. 61, 69-88 (1986) angegeben.

Als Hilfsstoffe gelten auch Zusätze von Basen und Säuren zur Steuerung der Löslichkeit eines Wirkstoffes (siehe beispielsweise K. Thoma et al., Pharm. Ind. 51, 98-101 (1989)).

Einzige Voraussetzung für die Eignung von Hilfsstoffen ist eine ausreichende Temperaturstabilität.

Unter Wirkstoffen im Sinne der Erfindung sind alle Stoffe mit einer physiologischen Wirkung zu verstehen, sofern sie sich unter den Verarbeitungsbedingungen nicht zersetzen. Es handelt sich insbesondere um pharmazeutische Wirkstoffe (für Mensch und Tier), Wirkstoffe für die Pflanzenbehandlung, Insektizide, Futter- und Nahrungsmittelwirkstoffe, Riechstoffe und Parfümöle. Die Wirkstoffmenge pro Dosiseinheit und die Konzentration können je nach Wirksamkeit und Freisetzungsgeschwindigkeit in weiten Grenzen variieren. Die einzige Bedingung ist, dass sie zur Erzielung der gewünschten Wirkung ausreichen. So kann die Wirkstoffkonzentration im Bereich von 0,1 bis 95, vorzugsweise von 20 bis 80, insbesondere 30 bis 70 Gew.-% liegen. Auch Wirkstoff-Kombinationen können eingesetzt werden. Wirkstoffe im Sinne der Erfindung sind auch Vitamine und Mineralstoffe. Zu den Vitaminen gehören die Vitamine der A-Gruppe, der B-Gruppe, worunter neben B₁, B₂, B₆ und B₁₂ sowie Nicotinsäure und Nicotinamid auch Verbindungen mit Vitamin B-Eigenschaften verstanden werden, wie z. B. Adenin, Cholin, Pantothensäure, Biotin, Adenylsäure, Folsäure, Orotsäure, Pangamsäure, Carnitin, p-Aminobenzoesäure, myo-Inosit und Liponsäure sowie Vitamin C, Vitamine der D-Gruppe, E-Gruppe, F-Gruppe, H-Gruppe, I- und J-Gruppe, K-Gruppe und P-Gruppe. Zu Wirkstoffen im Sinne der Erfindung gehören auch Peptidtherapeutika. Zu Pflanzenbehandlungsmitteln zählen z. B. Vinclozolin, Epoxiconazol und Quinmerac.

Das erfindungsgemäße Verfahren ist beispielsweise zur Verarbeitung folgender Wirkstoffe geeignet:

Acebutolol, Acetylcystein, Acetylsalicylsäure, Aciclovir, Alfacalcidol, Allantoin, Allopurinol, Alprazolam, Ambroxol, Amikacin, Amilorid, Aminoessigsäure, Amiodaron, Amitriptylin, Amlodipin, Amoxicillin, Ampicillin, Ascorbinsäure, Aspartam, Astemizol, Atenolol, Beclomethason, Benserazid, Benzalkonium-Hydrochlorid, Benzocain, Benzoesäure, Betamethason, Bezafibrat, Biotin, Biperiden, Bisoprolol, Bromazepam, Bromhexin, Bromocriptin, Budesonid, Bufexamac, Buflomedil, Buspiron, Coffein, Campher, Captopril, Carbamazepin, Carbidopa, Carboplatin, Cefachlor, Cefalexin, Cefadroxil, Cefazolin, Cefixim, Cefotaxim, Ceftazidim, Ceftriaxon, Cefuroxim, Chloramphenicol, Chlorhexidin, Chlor-pheniramin, Chlortalidon, Cholin, Cyclosporin, Cilastatin, Cimetidin, Ciprofloxacin, Cisapride, Cisplatin, Clarithromycin, Clävulansäure, Clomipramin, Clonazepam, Clonidin, Clotrimazol, Codein, Cholestyramin, Cromoglycinsäure, Cyanocobalamin, Cyproteron, Desogestrel, Dexamethason, Dexpanthenol, Dextromethorphan, Dextropropoxiphen, Diazepam, Diclofenac, Digoxin, Dihydrocodein, Dihydroergotamin, Dihydroergotoxin, Diltiazem, Diphenhydramin, Dipyridamol, Dipyron, Disopyramid, Domperidon, Dopamin, Doxycyclin, Enalapril, Ephedrin, Epinephrin, Ergocalciferol, Ergotamin, Erythromycin, Estradiol, Ethinylestradiol, Etoposid, Eucalyptus Globulus, Famotidin, Felodipin, Fenofibrat, Fenoterol, Fentanyl, Flavin-Mononucleotid, Fluconazol, Flunarizin, Fluorouracil, Fluoxetin, Flurbiprofen, Folinsäure, Furosemid, Gallopamil, Gemfibrozil, Gentamicin, Gingko Biloba, Glibenclamid, Glipizid, Clozapin, Glycyrrhiza glabra, Griseofulvin, Guaifenesin, Haloperidol, Heparin, Hyaluronsäure, Hydrochlorothiazid, Hydrocodon, Hydrocortison, Hydromorphon, Ipratropium-Hydroxid, Ibuprofen, Imipenem, Imipramin, Indomethacin, Iohexol, Iopamidol, Isosorbid-Dinitrat, Isosorbid-Mononitrat, Isotretinoin, Ketotifen, Ketoconazol, Ketoprofen, Ketorolac, Labetalol, Lactulose, Lecithin, Levocarnitin, Levodopa, Levoglutamid, Levonorgestrel, Levothyroxin, Lidocain, Lipase, Lisinopril, Loperamid, Lorazepam, Lovastatin, Medroxyprogesteron, Menthol, Methotrexat, Methyldopa, Methylprednisolon, Metoclopramid, Metoprolol, Miconazol, Midazolam, Minocyclin, Minoxidil, Misoprostol, Morphin, Multivitamin-Mischungen bzw. -Kombinationen und Mineralsalze, N-Methylephedrin, Naftidrofuryl, Naproxen, Neomycin, Nicardipin, Nicergolin, Nicotinamid, Nicotin, Nicotinsäure, Nifedipin, Nimodipin, Nitrazepam, Nitrendipin, Nizatidin, Norethisteron, Norfloxacin, Norgestrel, Nortriptylin, Nystatin, Ofloxacin, Omeprazol, Ondansetron, Pancreatin, Panthenol, Pantothensäure, Paracetamol, Penicillin G, Penicillin V, Pentoxifyllin, Phenobarbital, Phenoxymethylpenicillin, Phenylephrin, Phenylpropanolamin, Phenytoin, Piroxicam, Polymyxin B, Povidon-Iod, Pravastatin, Prazepam, Prazosin, Prednisolon, Prednison, Propafenon, Propranolol, Proxyphyllin, Pseudoephedrin, Pyridoxin, Quinidin, Ramipril, Ranitidin, Reserpin, Retinol, Riboflavin, Rifampicin, Rutosid, Saccharin, Salbutamol, Salcatonin, Salicylsäure, Selegilin, Simvastatin, Somatropin, Sotalol, Spironolacton, Sucralfat, Sulbactam, Sulfamethoxazol, Sulfasalazin, Sulpirid, Tamoxifen, Tegafur, Teprenon, Terazosin, Terbutalin, Terfenadin, Tetracyclin, Theophyllin, Thiamin, Ticlopidin, Timolol, Tranexamsäure, Tretinoin, Triamcinolon-Acetonid, Triamteren, Trimethoprim, Troxerutin, Uracil, Valproinsäure, Vancomycin, Verapamil, Vitamin E, Zidovudin.

Bevorzugte Wirkstoffe sind Ibuprofen (als Racemat, Enantiomer oder angereichertes Enantiomer), Ketoprofen, Flurbiprofen, Acetylsalicylsäure, Verapamil, Paracetamol, Nifedipin oder Captopril.

Zur Herstellung der festen Dosierungsformen wird ein plastisches Gemisch der Komponenten (Schmelze) bereitgestellt, das anschließend einem Formgebungsschritt unterzogen wird. Das Vermischen der Komponenten und die Bildung der Schmelze können auf unterschiedliche Weise erfolgen. Das Vermischen kann vor, während und/oder nach der Bildung der Schmelze erfolgen. Beispielsweise können die Komponenten zuerst vermischt und dann aufgeschmolzen oder gleichzeitig vermischt und aufgeschmolzen werden. Häufig erfolgt noch eine Homogenisierung des plastischen Gemisches, um eine hochdisperse Verteilung des Wirkstoffes zu erhalten.

Insbesondere bei Verwendung von empfindlichen Wirkstoffen hat es sich aber als bevorzugt erwiesen, zuerst das polymere Bindemittel, gegebenenfalls zusammen mit üblichen pharmazeutischen Additiven, aufzuschmelzen und vorzuvermischen und dann den (die) empfindlichen Wirkstoff(e) in "Intensivmischern" in plastischer Phase bei sehr kleinen Verweilzeiten einzumischen (Homogenisieren). Der (die) Wirkstoff(e) kann (können) dabei in fester Form oder als Lösung oder Dispersion eingesetzt werden.

Im Allgemeinen werden die Komponenten als solche in das Herstellungsverfahren eingesetzt. Sie können jedoch auch in flüssiger Form, d. h. als Lösung, Suspension oder Dispersion zur Anwendung kommen.

Als Lösungsmittel für die flüssige Form der Komponenten kommt in erster Linie Wasser oder ein mit Wasser mischbares, organisches Lösungsmittel oder ein Gemisch davon mit Wasser in Betracht. Brauchbare Lösungsmittel sind aber auch mit Wasser nicht mischbare oder mischbare, organische Lösungsmittel. Geeignete, mit Wasser mischbare Lösungsmittel sind insbesondere C₁-C₄-Alkanole, wie Ethanol, Isopropanol oder n-Propanol, Polyole, wie Ethylenglykol, Glycerin und Polyethylenglykole. Geeignete, mit Wasser nicht mischbare Lösungsmittel sind Alkane, wie Pentan oder Hexan, Ester, wie Ethylacetat oder Butylacetat, chlorierte Kohlenwasserstoffe, wie Methylenchlorid und aromatische Kohlenwasserstoffe, wie Toluol und Xylol. Ein weiteres brauchbares Lösungsmittel ist flüssiges CO₂.

Welches Lösungsmittel im Einzelfall verwendet wird, hängt von der aufzunehmenden Komponente und deren Eigenschaften ab. Beispielsweise kommen pharmazeutische Wirkstoffe häufig in Form eines Salzes, das im Allgemeinen wasserlöslich ist, zur Anwendung. Wasserlösliche Wirkstoffe können daher als wässrige Lösung eingesetzt werden oder vorzugsweise in die wässrige Lösung oder Dispersion des Bindemittels aufgenommen werden. Entsprechendes gilt für Wirkstoffe, die in einem der genannten Lösungsmittel löslich sind, wenn die flüssige Form der zur Anwendung kommenden Komponenten auf einem organischen Lösungsmittel basiert.

Gegebenenfalls kann an die Stelle des Aufschmelzens ein Lösen, Suspendieren oder Dispergieren in den oben genannten Lösungsmitteln, falls erwünscht und/oder erforderlich unter Zusatz geeigneter Hilfsstoffe, wie z. B. Emulgatoren, treten. Das Lösungsmittel wird dann im Allgemeinen unter Bildung der Schmelze in einer geeigneten Apparatur, z. B. einem Extruder, entfernt. Im Folgenden soll dies von dem Begriff Vermischen umfasst werden.

Das Aufschmelzen und/oder Vermischen erfolgt in einer für diesen Zweck üblichen Vorrichtung. Besonders geeignet sind Extruder oder gegebenenfalls beheizbare Behälter mit Rührwerk, z. B. Kneter, (wie der unten noch erwähnten Art).

Als Mischapparat sind insbesondere solche Vorrichtungen brauchbar, die in der Kunststofftechnologie zum Mischen eingesetzt werden. Geeignete Vorrichtungen sind beispielsweise beschrieben in "Mischen beim Herstellen und Verarbeiten von Kunststoffen", H. Pahl, VDI-Verlag, 1986. Besonders geeignete Mischapparaturen sind Extruder und dynamische und statische Mischer, sowie Rührkessel, einwellige Rührwerke mit Abstreifvorrichtungen, insbesondere sogenannte Pastenrührwerke, mehrwellige Rührwerke, insbesondere PDSM-Mischer, Feststoffmischer sowie vorzugsweise Misch-Knetreaktoren (z. B. ORP, CRP, AP, DTB der Firma List oder Reactotherm der Firma Krauss-Maffei oder Ko-Kneter der Fa. Buss), Doppelmuldenkneter (Trogmischer) und Stempelkneter (Innenmischer) oder Rotor/Stator-Systeme (z. B. Dispax der Firma IKA).

Bei empfindlichen Wirkstoffen erfolgt vorzugsweise zunächst das Aufschmelzen des polymeren Bindemittels in einem Extruder und anschließend das Zumischen des Wirkstoffs in einem Misch-Knetreaktor. Bei weniger empfindlichen Wirkstoffen kann man dagegen zum intensiven Dispergieren des Wirkstoffs ein Rotor/Stator-System einsetzen.

Das Beschicken der Mischvorrichtung erfolgt je nach deren Konzeption kontinuierlich oder diskontinuierlich in üblicher Weise. Pulverförmige Komponenten können im freien Zulauf, z. B. über eine Differentialdosierwaage eingeführt werden. Plastische Massen können direkt aus einem Extruder eingespeist oder über eine Zahnradpumpe, die insbesondere bei hohen Viskositäten und hohen Drüken von Vorteil ist, zugespeist werden. Flüssige Medien können über ein geeignetes Pumpenaggregat zudosiert werden.

Das durch Vermischen und/oder Aufschmelzen des Bindemittels, des Wirkstoffes und gegebenenfalls des Additivs oder der Additive erhaltene Gemisch ist teigig bis zähflüssig (thermoplastisch) oder flüssig und daher extrudierbar. Die Glasübergangstemperatur des Gemisches liegt unter der Zersetzungstemperatur aller in dem Gemisch enthaltenen Komponenten. Das Bindemittel soll vorzugsweise in physiologischer Umgebung löslich oder quellbar sein.

Die Verfahrensschritte Vermischen und Aufschmelzen können in derselben Apparatur oder in zwei oder mehreren getrennt arbeitenden Vorrichtungen ausgeführt werden. Die Zubereitung einer Vormischung kann in einer der oben beschriebenen üblichen Mischvorrichtungen durchgeführt werden. Eine solche Vormischung kann dann direkt, z. B. in einen Extruder, eingespeist und anschließend ggf. unter Zusatz weiterer Komponenten extrudiert werden.

Das erfindungsgemäße Verfahren erlaubt es, als Extruder Einschneckenmaschinen, kämmende Schneckenmaschinen oder auch Mehrwellextruder, insbesondere Zweischnecken-Extruder, gleichsinnig oder gegensinnig drehend und gegebenenfalls mit Knetscheiben ausgerüstet, einzusetzen. Wenn bei der Extrusion ein Lösungsmittel verdampft werden muss, sind die Extruder im Allgemeinen mit einem Verdampfungsteil ausgerüstet. Besonders bevorzugt sind Extruder der ZKS-Baureihe von Werner u. Pfleiderer.

Erfindungsgemäß können auch mehrschichtige pharmazeutische Formen durch Koextrusion hergestellt werden, wobei mehrere Gemische aus den oben beschriebenen Komponenten bei der Extrusion so in einem Werkzeug zusammengeführt werden, dass sich der gewünschte Schichtaufbau der mehrschichtigen pharmazeutischen Form ergibt. Vorzugsweise verwendet man verschiedene Bindemittel für verschiedene Schichten.

Mehrschichtige Arzneiformen umfassen vorzugsweise zwei oder drei Schichten. Sie können in offener oder geschlossener Form vorliegen, insbesondere als offene oder geschlossene Mehrschichttabletten.

Wenigstens eine der Schichten enthält wenigstens einen pharmazeutischen Wirkstoff. Es ist auch möglich, einen weiteren Wirkstoff in eine andere Schicht aufzunehmen. Dies hat den Vorteil, dass zwei miteinander unverträgliche Wirkstoffe verarbeitet werden können oder dass die Freisetzungscharakteristik des Wirkstoffes gesteuert werden kann.

Das Ausformen erfolgt durch Koextrusion, wobei die Gemische aus den einzelnen Extrudern oder anderen Aggregaten in ein gemeinsames Koextrusionswerkzeug geführt und ausgetragen werden. Die Form der Koextrusionswerkzeuge richtet sich nach der gewünschten pharmazeutischen Form. Beispielsweise sind Werkzeuge mit ebenem Austrittsspalt, sogenannte Breitschlitzwerkzeuge, und Werkzeuge mit kreisringspaltförmigem Austrittsquerschnitt geeignet. Die Düsenauslegung erfolgt dabei in Abhängigkeit von dem zur Anwendung kommenden polymeren Bindemittel und der gewünschten pharmazeutischen Form.

Das erhaltene Gemisch ist vorzugsweise lösungsmittelfrei, d.h. es enthält weder Wasser noch ein organisches Lösungsmittel.

Das plastische Gemisch wird in der Regel einer abschließenden Formgebung unterzogen. Dabei kann eine Vielzahl von Formen, je nach Werkzeug und Art der Formung, erzeugt werden. Beispielsweise lässt sich bei Verwendung eines Extruders der extrudierte Strang zwischen einem Band und einer Walze, zwischen zwei Bändern oder zwischen zwei Walzen, wie in der EP-A-358 105 beschrieben, oder durch Kalandrierung in einem Kalander mit zwei Formwalzen, siehe beispielsweise EP-A-240 904, formen. Durch Extrusion und Heiß- oder Kaltabschlag des Stranges können weitere Formen erhalten werden, beispielsweise kleinteilige und gleichmäßig geformte Granulate. Die Heißgranulierung führt in der Regel zu linsenförmigen Dosierungsformen (Tabletten) mit einem Durchmesser von 1 bis 10 mm, während die Kaltgranulierung normalerweise zu zylinderförmigen Produkten mit einem Verhältnis von Länge zu Durchmesser von 1 bis 10 und einem Durchmesser von 0,5 bis 10 mm führt. So können einschichtige, bei Anwendung der Koextrusion aber auch offene oder geschlossene, mehrschichtige Dosierungsformen hergestellt werden, beispielsweise Oblongtabletten, Dragees, Pastillen und Pellets. Die erhaltenen Granulate können anschließend auch zu Pulver gemahlen und in üblicher Weise zu Tabletten verpresst werden. Mikropastillen können durch das Rotoform-Sandvik-Verfahren hergestellt werden. Diese Dosierungsformen können in einem nachgeschalteten Verfahrensschritt nach üblichen Methoden gerundet und/oder mit einem Coating versehen werden. Geeignete Materialien für Filmüberzüge sind z. B. Polyacrylate, wie die Eudragit-Typen, Celluloseester, wie die Hydroxypropylcellulosephthalate, sowie Celluloseether, wie Ethylcellulose, Hydroxypropylmethylcellulose oder Hydroxypropylcellulose.

Im Einzelnen kann es zur Ausbildung von festen Lösungen kommen. Der Begriff "feste Lösungen" ist dem Fachmann geläufig, beispielsweise aus der eingangs zitierten Literatur. In festen Lösungen von Wirkstoffen in Polymeren liegt der Wirkstoff molekulardispers im Polymer vor.

Das folgende Beispiel soll das erfindungsgemäße Verfahren veranschaulichen, ohne es jedoch zu beschränken.

### Beispiel 1

520 g eines Polyamids aus 16,67 mol-% Natrium-5-Sulfoisophthalsäure, 16,67 mol-% Isophthalsäure, 33,33 mol-% Hexamethylendiamin und 33,33 % ε-Caprolactam (K-Wert 21,0; 1%ig in Dimethylformamid; Mₙ (Endgruppenanalyse): 6000-7000; T_{g} = 149 °C) werden mit 480 g Verapamil-Hydrochlorid unter den nachfolgend angegebenen Bedingungen extrudiert und zu 500 mg-Oblong-Tabletten nach dem in der EP-A-240 904 beschriebenen Verfahren kalandriert.

| | |
|---|---|
| Schuss 1 | 94 °C |
| Schuss 2 | 150 °C |
| Schuss 3 | 123 °C |
| Schuss 4 | 100 °C |
| Schuss 5 | 81 °C |
| Düse | 79 °C |

Die Freisetzung nach 8 h betrug 10 % [Paddlemodell nach USP (pH change)].

## Patentansprüche

1. Verfahren zur Herstellung von festen Dosierungsformen durch Vermischen von mindestens einem polymeren Bindemittel, gegebenenfalls mindestens einem Wirkstoff und gegebenenfalls üblichen Additiven unter Bildung eines plastischen Gemisches und Formgebung, dadurch gekennzeichnet, dass man als polymeres Bindemittel sulfonatgruppentragende Polyamide verwendet.

2. Verfahren nach Anspruch 1, dadurch gekennzeichnet, dass das sulfonatgruppentragende Polyamid erhältlich ist aus
A1) 0 bis 90 mol-%, vorzugsweise 0 bis 50 mol-%, besonders bevorzugt 0 bis 45 mol-%, mindestens einer Monoaminocarbonsäure, deren Lactam oder Monoaminocarbonsäure-/-lactam-Gemischen,
A2) 5 bis 50 mol-%, vorzugsweise 45 bis 50 mol-%, besonders bevorzugt 47,5 bis 50 mol-%, mindestens eines primären oder sekundären Diamins,
A3) 0,5 bis 49,5 mol-%, vorzugsweise 5 bis 35 mol-%, besonders bevorzugt 10 bis 30 mol-%, mindestens einer sulfonatgruppentragenden Dicarbonsäure und gegebenenfalls
A4) 0,5 bis 49,5 mol-%, vorzugsweise 15 bis 45 mol-%, besonders bevorzugt 20 bis 40 mol-%, mindestens einer weiteren Dicarbonsäure,
wobei die Summe der molaren Anteile der Monomeren A3) und A4) im Wesentlichen dem molaren Anteil des Monomeren A2) entspricht.

3. Verfahren nach einem der Ansprüche 1 oder 2, dadurch gekennzeichnet, dass die Monomere A2) mindestens zwei verschiedene Diamine umfassen.

4. Verfahren nach einem der vorhergehenden Ansprüche, dadurch gekennzeichnet, dass die sulfonatgruppentragende Dicarbonsäure das Natriumsalz der 5-Sulfoisophthalsäure ist.

5. Verfahren nach einem der vorhergehenden Ansprüche, dadurch gekennzeichnet, dass die Bildung des plastischen Gemisches durch Vermischen und/oder Aufschmelzen der Komponenten in einem Extruder erfolgt.

6. Verfahren nach einem der Ansprüche 1 bis 5 zur Herstellung von pharmazeutischen wirkstoffhaltigen Dosierungsformen.

7. Verfahren nach einem der Ansprüche 1 bis 5 zur Herstellung von Pflanzenbehandlungsmitteln.

8. Verfahren nach einem der Ansprüche 1 bis 5 zur Herstellung von Futtermittelzusatzstoffen und -zusätzen.

9. Verfahren nach einem der Ansprüche 1 bis 5 zur Herstellung von Nahrungsmittelzusätzen.

10. Feste Dosierungsformen, erhältlich nach einem Verfahren gemäß einem der Ansprüche 1 bis 9.
